# EUROPÄISCHE PATENTANMELDUNG

(11) **EP 1 118 609 A2**
(43) Veröffentlichungstag der Anmeldung: **25.07.2001**
(21) Anmeldenummer: 01100442.1
(22) Anmeldetag: 08.01.2001
(51) Int. Cl.: C07C 251/48, A01N 37/50

(54) **Iminooxisubstituierte Benzylphenylether, Verfahren und Zwischenprodukte zu ihrer Herstellung, sie enthaltende Mittel sowie ihre Verwendung zur Bekämpfung von Schadpilzen**

(30) Priorität: 21.01.2000 DE 10002661
(71) Anmelder: BASF AKTIENGESELLSCHAFT, 67056 Ludwigshafen (DE)
(72) Erfinder: Grammenos, Wassilios, Dr., 67063 Ludwigshafen (DE); Sauter, Hubert, Dr., 68167 Mannheim (DE); Gypser, Andreas, Dr., 68159 Mannheim (DE); Gewehr, Markus, Dr., 56288 Kastellaun (DE); Tormo i Blasco, Jordi, Dr., 67117 Limburgerhof (DE); Ptock, Arne, Dr., 67065 Ludwigshafen (DE); Müller, Bernd, Dr., 67227 Frankenthal (DE); Cullmann, Oliver, Dr., 68199 Mannheim (DE); Grote, Thomas, Dr., 67157 Wachenheim (DE); Ammermann, Eberhard, Dr., 64646 Heppenheim (DE); Strathmann, Siegfried, Dr., 67117 Limburgerhof (DE); Lorenz, Gisela, Dr., 67434 Neustadt (DE)
(74) Vertreter: Fitzner, Ulrich, Dr.

(57) **Zusammenfassung**

Iminooxisubstituierte Benzylphenylether, Verfehren und Zwischenprodukte zu ihrer Herstellung, sie enthaltende Mittel sowie ihre Verwendung zur Bekämpfung von Schadpilzen

Zusammenfassung

Iminooxisubstituierte Benzylphenylether der Formel I, 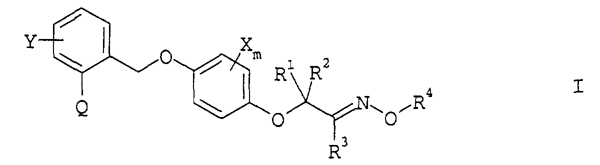 in der die Substituenten und der Index die folgenden Bedeutungen haben:
- Y: H, CH₃, F oder Cl
- Q: C(=CHCCH₃)-COOCH₃, C(=CHCH₃)-COOCH₃, C(=NOCH₃)-COOCH₃, C(=NOCH₃)-CONHCH₃ oder N(-OCH₃)-COOCH₃;
- X: Wasserstoff, Halogen, Alkyl, Alkoxy oder CF₃;
- m: 1 oder 2, wobei die Reste X verschieden sein können, wenn m = 2 ist;
- R¹: Alkyl und
- R²: Wasserstoff oder Alkyl; oder
- R¹ und R²: gemeinsam Cyclopropyl, Cyclopentyl oder Cyclohexyl;
- R³: Alkyl oder CF₃; und
- R⁴: Alkyl, Alkenyl, Alkinyl, Halogenalkyl, Halogenalkenyl oder Halogenalkinyl;

Verfahren und Zwischenprodukte zu ihrer Herstellung, sie enthaltende Mittel und ihre Verwendung.

## Beschreibung

Die vorliegende Erfindung betrifft iminooxisubstituierte Benzylphenylether der Formel I, 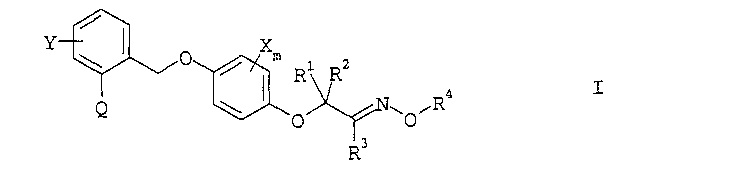 in der die Substituenten und der Index die folgenden Bedeutungen haben:
- Y: H, CH₃, F oder Cl;
- Q: C(=CHOCH₃) -COOCH₃, C(=CHCH₃) -COOCH₃, C(=NOCH₃) -COOCH₃, C(=NOCH₃) -CONHCH₃ oder N(-OCH₃) -COOCH₃;
- X: Wasserstoff, Halogen, C₁-C₄-Alkyl, C₁-C₄-Alkoxy oder CF₃;
- m: 1 oder 2, wobei die Reste X verschieden sein können, wenn m = 2 ist;
- R¹: C₁-C₄-Alkyl und
- R²: Wasserstoff oder C₁-C₄-Alkyl; oder
- R¹ und R²: gemeinsam Cyclopropyl, Cyclopentyl oder Cyclohexyl;
- R³: C₁-C₆-Alkyl oder CF₃; und
- R⁴: C₁-C₄-Alkyl, C₃-C₄-Alkenyl, C₃-C₄-Alkinyl, C₁-C₄-Halogenalkyl, C₃-C₄-Halogenalkenyl oder C₃-C₄-Halogenalkinyl;
sowie deren Salze.

Zusätzlich betrifft die Erfindung Verfahren und Zwischenprodukte zur Herstellung der Verbindungen I, sowie Mittel und die Verwendung der Verbindungen I zur Bekämpfung von Schadpilzen.

Phenylcarbamate mit orthoständiger Phenoxymethylen-Gruppe werden in WO-A 93/15046 offenbart. α-Phenylacrylsäure- und α-Phenyl-α-methoximinoessigsäurederivate mit orthoständiger Phenoxymethylen-Gruppierung sind aus EP-A 253 213, EP-A 280 185, EP-A 386 561, EP-A 477 631, EP-A 513 580 und EP-A 579 124 bekannt. Einige der dort beschriebenen Verbindungen tragen einen Oximinorest an der Phenoxygruppierung.

Die in den vorstehend genannten Schriften beschriebenen Verbindungen sind als Pflanzenschutzmittel gegen Schadpilze geeignet.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die substituierten Benzylphenylether der Formel I gefunden. Weiterhin wurden Zwischenprodukte und Verfahren zur Herstellung der Verbindungen I, sowie die Verwendung der Verbindungen I und diese enthaltene Mittel zur Bekämpfung von Schadpilzen gefunden.

Die Verbindungen der Formel I unterscheiden sich von den aus den oben genannten Schriften bekannten Verbindungen in der Substitution der Phenoxygruppe durch den über Sauerstoff gebundenen Oximinorest. Die Verbindungen der Formel I weisen eine gegenüber den bekannten Verbindungen erhöhte Wirksamkeit gegen Schadpilze auf.

Verbindungen der Formel I erhält man beispielsweise ausgehend von Hydroxiverbindungen der Formel II, 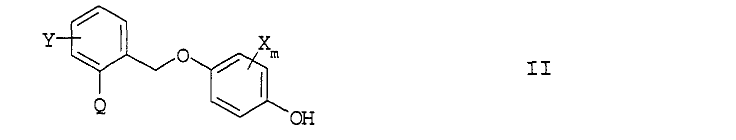 die durch Oxidation von Acetylverbindungen der Formel II.1 erhältlich sind.

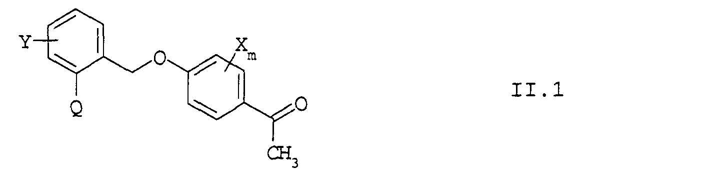

Die Oxidation erfolgt üblicherweise bei Temperaturen von -20 °C bis 60 °C, vorzugsweise 0 °C bis 30 °C, in einem inerten organischen Lösungsmittel oder Wasser in Gegenwart einer Base oder eines Puffers, wie beispielsweise Na₂HPO₄ [vgl. Synthesis, S.63 (1991); Heterocycles, S.819 (1993); Tetrahedron, S.3197 (1995)].

Als Oxidationsmittel kommen beispielsweise Wasserstoffperoxid, m-Chlorperbenzoesäure, Peressigsäure, H₂O₂ x BF₃, Peroxytrifluoressigsäure oder K₂S₂O₇/H₂SO₄ in Betracht.

Als Lösungsmittel kommen allgemein aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, als Basen anorganische Verbindungen wie Alkalimetall- und Erdalkalimetallhydroxide wie Lithiumhydroxid, Natriumhydroxid, Kaliumhydroxid und Calziumhydroxid in Betracht. Besonders bevorzugt ist Natriumhydroxid.

Die Base wird im allgemeinen in katalytischen Mengen eingesetzt, sie kann aber auch äquimolar oder im Überschuß verwendet werden.

Die Oxidation der Acetylverbindungen II.1 kann auch zweistufig durchgeführt werden. Sie führt primär zu Acetyloxiverbindungen der Formel II.2, aus der direkt oder, falls gewünscht in einem separaten Schritt, unter basischen Bedingungen die Hydroxiverbindung II freigesetzt werden kann.

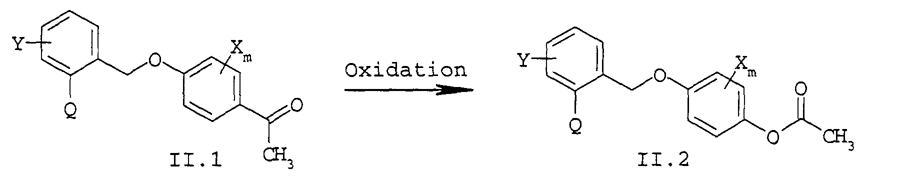

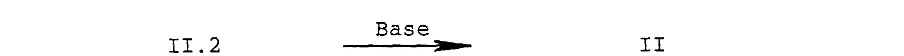

Verbindungen der Formel I, in der Q für C(=NOCH₃)-CONHCH₃ steht, werden bevorzugt so hergestellt, daß man in der vorstehend skizzierten Reaktionsfolge von Verbindungen II.1 ausgeht, in denen Q für C(=NOCH₃)-COOCH₃ steht und im zweiten Schritt Methylamin als Base verwendet.

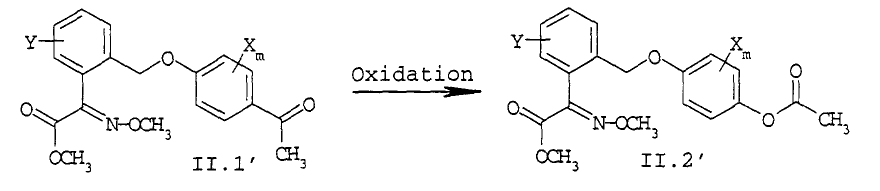

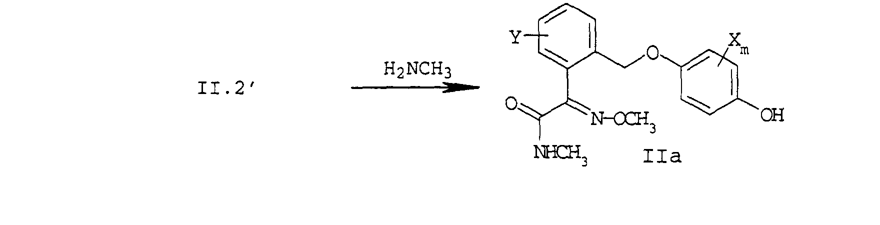

Die für die Herstellung der Verbindungen I benötigten Ausgangsstoffe II.1 sind in der Literatur bekannt [vgl. EP-A 513 580; EP-A 621 264] oder können gemäß der zitierten Literatur hergestellt werden.

Hydroxiverbindungen der Formel II werden mit Ketoverbindungen der Formel III zu Verbindungen der Formel IV umgesetzt.

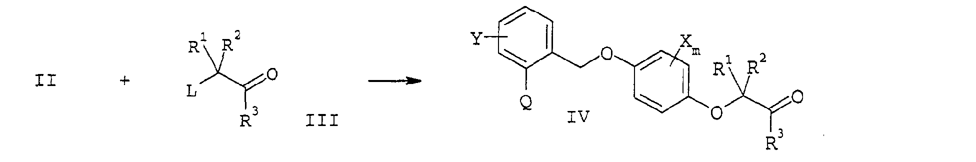

In Formel III steht L für eine nucleophil austauschbare Gruppe wie Halogen, bevorzugt Chlor. Die Umsetzung erfolgt üblicherweise bei Temperaturen von -10 °C bis 120 °C, vorzugsweise 20 °C bis 90 °C, in einem inerten organischen Lösungsmittel in Gegenwart einer Base [vgl. DE-A 38 27 222].

Geeignete Lösungsmittel sind Ether, Nitrile, Ketone wie Aceton, Methylethylketon, Diethylketon und tert.-Butylmethylketon, sowie Dimethylsulfoxid, Dimethylformamid und Dimethylacetamid, besonders bevorzugt Dimethylformamid, Aceton und Dimethylacetamid. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetallhydride, Alkalimetallamide, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium-tert.-Butanolat und Dimethoxymagnesium in Betracht. Besonders bevorzugt werden Natriumhydrogencarbonat, Kaliumcarbonat, Natriummethanolat und Natriumhydroxid.

Die Basen werden im allgemeinen in katalytischen Mengen eingesetzt, sie können aber auch äquimolar oder im Überschuß verwendet werden.

Die Edukte werden im allgemeinen in äquimolaren Mengen miteinander umgesetzt. Es kann für die Ausbeute vorteilhaft sein, III in einem Überschuß bezogen auf II einzusetzen.

Ketoverbindungen der Formel III sind bekannt. Sie sind z.T. kommerziell erhältlich oder nach bekannten Methoden herstellbar [Synthesis, S.595 (1990); DE-A 27 16 895; Can.J.Chem., S.2387 (1972); Tetrahedron S.5191 (1970); Synthesis S.189-191 (1977); EP-A 297 383; EP-A 298 332; Bull.Soc.Chim.Fr. S.2732 (1973); J.Org.Chem. S.8320 (1995); J.Organomet.Chem. S.325 (1975)].

Die Umsetzung von IV zu den Verbindungen der Formel I erfolgt mit Aminoverbindungen V, die bevorzugt als wässrige Lösung oder in Form ihrer Säureadditionssalze, z.B. als Hydrochloride, verwendet werden.

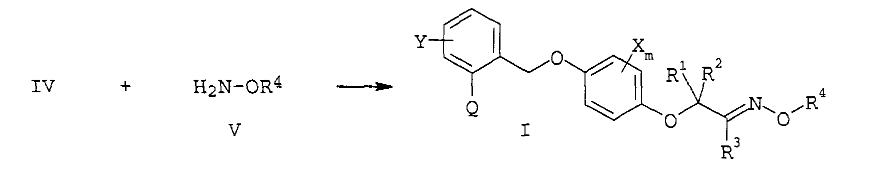

Diese Umsetzung erfolgt üblicherweise bei Temperaturen von 0 °C bis 120 °C, vorzugsweise 20 °C bis 80 °C, in einem inerten organischen Lösungsmittel in Gegenwart einer Base [vgl. EP-A 386 561].

Geeignete Lösungsmittel sind aromatische Kohlenwasserstoffe wie Toluol oder Alkohole wie Methanol oder Ethanol, besonders bevorzugt Methanol. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, außerdem organische Basen, z.B. tertiäre Amine wie Trimethylamin, Triethylamin, Tri-isopropylamin in Betracht. Besonders bevorzugt werden Natriumhydroxid, Kaliumcarbonat und Triethylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, V in einem Überschuß bezogen auf IV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Bei den in den vorstehenden Formeln angegebenen Definitionen der Symbole wurden Sammelbegriffe verwendet, die allgemein repräsentativ für die folgenden Substituenten stehen:
**Halogen**: Fluor, Chlor, Brom und Jod;
**Alkyl**: gesättigte, geradkettige oder verzweigte Kohlenwasserstoffreste mit 1 bis 4 oder 6 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy**: geradkettige oder verzweigte Alkylgruppen mit 1 bis 4 Kohlenstoffatomen (wie vorstehend genannt), welche über ein Sauerstoffatom (-O-) an das Gerüst gebunden sind;
**Alkenyl:** ungesättigte, geradkettige oder verzweigte Kohlenwasserstoffreste mit 3 oder 4 Kohlenstoffatomen und einer Doppelbindung in einer beliebigen Position, wie 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl;
**Halogenalkenyl**: ungesättigte, geradkettige oder verzweigte Kohlenwasserstoffreste mit 3 oder 4 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl**: geradkettige oder verzweigte Kohlenwasserstoffgruppen mit 3 oder 4 Kohlenstoffatomen und einer Dreifachbindung in einer beliebigen Position, wie 1-Propinyl, 2-Propinyl, 1-Butinyl, 2-Butinyl, 3-Butinyl, 1-Methyl-2-propinyl;
**Halogenalkinyl**: ungesättigte, geradkettige oder verzweigte Kohlenwasserstoffreste mit 3 oder 4 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können.

Im Hinblick auf die bestimmungsgemäße Verwendung der Benzylphenylether der Formel I sind die folgenden Bedeutungen der Substituenten, und zwar jeweils für sich allein oder in Kombination, besonders bevorzugt:

Verbindungen der Formel I, in denen X in der 2-Position steht und nicht Wasserstoff bedeutet, sind bevorzugt.

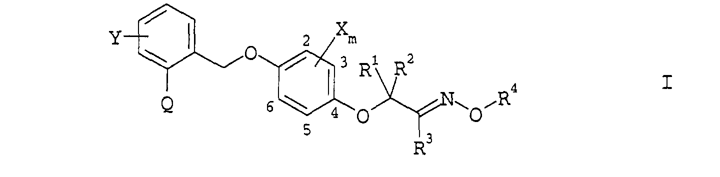

Verbindungen der Formel Ia sind besonders bevorzugt.

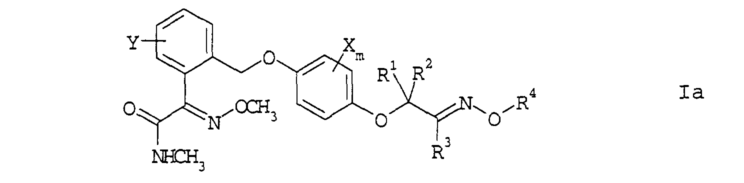

Daneben sind auch Verbindungen der Formel Ib bevorzugt.

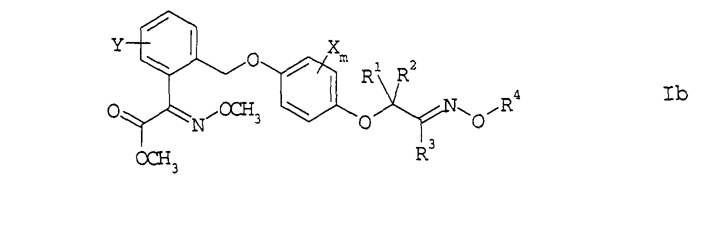

Außerdem sind auch Verbindungen der Formel Ic bevorzugt.

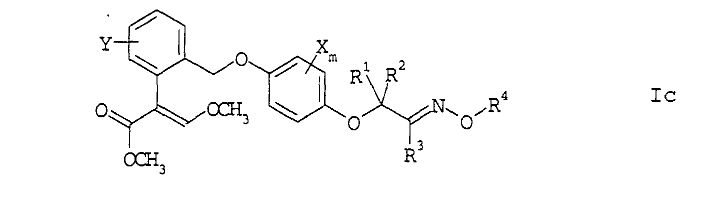

Gleichermaßen sind auch Verbindungen der Formel Id bevorzugt.

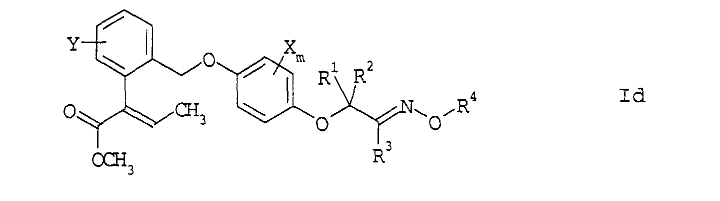

Weiterhin sind Verbindungen der Formel Ie bevorzugt.

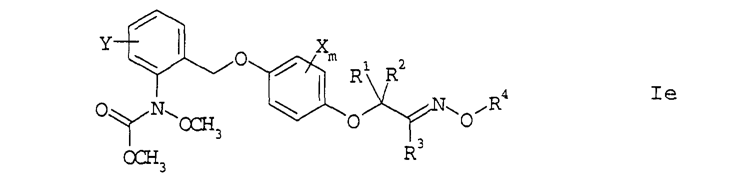

Insbesondere werden Verbindungen I' bevorzugt.

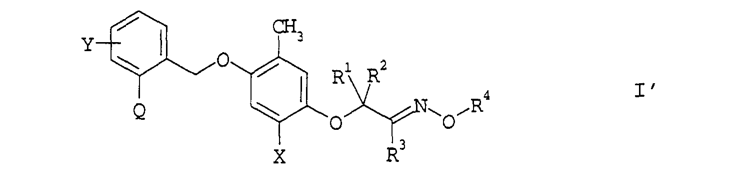

Gleichermaßen besonders bevorzugt sind Verbindungen I', in denen X für Wasserstoff steht.

Außerdem werden Verbindungen I besonders bevorzugt, in denen Q für C(=NOCH₃)-COOCH₃, C(=NOCH₃)-CONHCH₃ oder N(-OCH₃)-COOCH₃ steht.

Gleichermaßen besonders bevorzugt sind Verbindungen I', in denen Y in der 6-Position steht.

Außerdem werden Verbindungen I besonders bevorzugt, in denen Y für Wasserstoff steht.

Daneben werden Verbindungen I" besonders bevorzugt

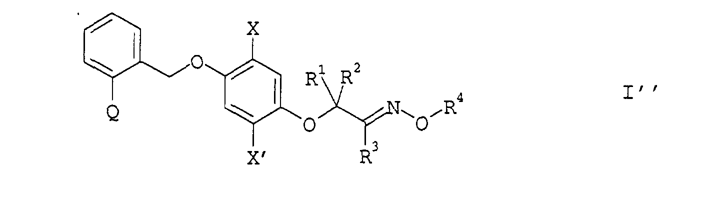

Insbesondere werden auch Verbindungen I' bevorzugt, in denen X für Wasserstoff, Methyl und Chlor steht.

Außerdem werden Verbindungen I' besonders bevorzugt, in denen R¹ für Methyl, Ethyl und iso-Propyl und R² für Wasserstoff steht.

Gleichermaßen besonders bevorzugt sind Verbindungen I', in denen R¹ und R² gemeinsam für Dimethyl und Cyclopropyl steht.

Daneben werden Verbindungen I' besonders bevorzugt, in denen R³ für Methyl und Ethyl steht.

Desweiteren werden Verbindungen I' besonders bevorzugt, in denen R⁴ für C₁-C₄-Alkyl steht.

Die besonders bevorzugten Ausführungsformen der Zwischenprodukte in Bezug auf die Variablen entsprechen denen der Reste Y, Q, Xₘ, R¹, R², R³ und R⁴ der Formel I.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I, in denen Q für C(=NOCH₃)-CONHCH₃, R¹ für Methyl und R² für Wasserstoff steht und die Kombination der Reste Y, X, X', R³ und R⁴ für eine Verbindung jeweils einer Zeile der Tabelle A entspricht

### Tabelle 2

Verbindungen der Formel I, in denen Q für C(=NOCH₃)-COOCH₃, R¹ für Methyl und R² für Wasserstoff steht und die Kombination der Reste Y, X, X', R³ und R⁴ für eine Verbindung jeweils einer Zeile der Tabelle A entspricht

### Tabelle 3

Verbindungen der Formel I, in denen Q für C(=CHOCH₃)-COOCH₃, R¹ für Methyl und R² für Wasserstoff steht und die Kombination der Reste Y, X, X', R³ und R⁴ für eine Verbindung jeweils einer Zeile der Tabelle A entspricht

### Tabelle 4

Verbindungen der Formel I, in denen Q für C(=CHCH₃)-COOCH₃, R¹ für Methyl und R² für Wasserstoff steht und die Kombination der Reste Y, X, X', R³ und R⁴ für eine Verbindung jeweils einer Zeile der Tabelle A entspricht

### Tabelle 5

Verbindungen der Formel I, in denen Q für N(-OCH₃)-COOCH₃, R¹ für Methyl und R² für Wasserstoff steht und die Kombination der Reste Y, X, X', R³ und R⁴ für eine Verbindung jeweils einer Zeile der Tabelle A entspricht

### Tabelle 6

Verbindungen der Formel I, in denen Q für C(=NOCH₃)-CONHCH₃, R¹ für Methyl und R² für Methyl steht und die Kombination der Reste Y, X, X', R³ und R⁴ für eine Verbindung jeweils einer Zeile der Tabelle A entspricht

### Tabelle 7

Verbindungen der Formel I, in denen Q für C(=NOCH₃)-COOCH₃, R¹ für Methyl und R² für Methyl steht und die Kombination der Reste Y, X, X', R³ und R⁴ für eine Verbindung jeweils einer Zeile der Tabelle A entspricht

### Tabelle 8

Verbindungen der Formel I, in denen Q für C(=CHOCH₃)-COOCH₃, R¹ für Methyl und R² für Methyl steht und die Kombination der Reste Y, X, X', R³ und R⁴ für eine Verbindung jeweils einer Zeile der Tabelle A entspricht

### Tabelle 9

Verbindungen der Formel I, in denen Q für C(=CHCH₃)-COOCH₃, R¹ für Methyl und R² für Methyl steht und die Kombination der Reste Y, X, X', R³ und R⁴ für eine Verbindung jeweils einer Zeile der Tabelle A entspricht

### Tabelle 10

Verbindungen der Formel I, in denen Q für N(-OCH₃)-COOCH₃, R¹ für Methyl und R² für Methyl steht und die Kombination der Reste Y, X, X', R³ und R⁴ für eine Verbindung jeweils einer Zeile der Tabelle A entspricht

### Tabelle 11

Verbindungen der Formel I, in denen Q für C(=NOCH₃)-CONHCH₃, R¹ für Ethyl und R² für Wasserstoff steht und die Kombination der Reste Y, X, X', R³ und R⁴ für eine Verbindung jeweils einer Zeile der Tabelle A entspricht

### Tabelle 12

Verbindungen der Formel I, in denen Q für C(=NOCH₃)-COOCH₃, R¹ für Ethyl und R² für Wasserstoff steht und die Kombination der Reste Y, X, X', R³ und R⁴ für eine Verbindung jeweils einer Zeile der Tabelle A entspricht

### Tabelle 13

Verbindungen der Formel I, in denen Q für C(=CHOCH₃)-COOCH₃, R¹ für Ethyl und R² für Wasserstoff steht und die Kombination der Reste Y, X, X', R³ und R⁴ für eine Verbindung jeweils einer Zeile der Tabelle A entspricht

### Tabelle 14

Verbindungen der Formel I, in denen Q für C(=CHCH₃)-COOCH₃, R¹ für Ethyl und R² für Wasserstoff steht und die Kombination der Reste Y, X, X', R³ und R⁴ für eine Verbindung jeweils einer Zeile der Tabelle A entspricht

### Tabelle 15

Verbindungen der Formel I, in denen Q für N(-OCH₃)-COOCH₃, R¹ für Ethyl und R² für Wasserstoff steht und die Kombination der Reste Y, X, X', R³ und R⁴ für eine Verbindung jeweils einer Zeile der Tabelle A entspricht

### Tabelle 16

Verbindungen der Formel I, in denen Q für C(=NOCH₃)-CONHCH₃, R¹ für iso-Propyl und R² für Wasserstoff steht und die Kombination der Reste Y, X, X', R³ und R⁴ für eine Verbindung jeweils einer Zeile der Tabelle A entspricht

### Tabelle 17

Verbindungen der Formel I, in denen Q für C(=NOCH₃)-COOCH₃, R¹ für iso-Propyl und R² für Wasserstoff steht und die Kombination der Reste Y, X, X', R³ und R⁴ für eine Verbindung jeweils einer Zeile der Tabelle A entspricht

### Tabelle 18

Verbindungen der Formel I, in denen Q für C(=CHOCH₃)-COOCH₃, R¹ für iso-Propyl und R² für Wasserstoff steht und die Kombination der Reste Y, X, X', R³ und R⁴ für eine Verbindung jeweils einer Zeile der Tabelle A entspricht

### Tabelle 19

Verbindungen der Formel I, in denen Q für C(=CHCH₃)-COOCH₃, R¹ für iso-Propyl und R² für Wasserstoff steht und die Kombination der Reste Y, X, X', R³ und R⁴ für eine Verbindung jeweils einer Zeile der Tabelle A entspricht

### Tabelle 20

Verbindungen der Formel I, in denen Q für N(-OCH₃)-COOCH₃, R¹ für iso-Propyl und R² für Wasserstoff steht und die Kombination der Reste Y, X, X', R³ und R⁴ für eine Verbindung jeweils einer Zeile der Tabelle A entspricht

### Tabelle 21

Verbindungen der Formel I, in denen Q für C(=NOCH₃)-CONHCH₃, R¹ und R² gemeinsam für Cyclopropyl stehen und die Kombination der Reste Y, X, X', R³ und R⁴ für eine Verbindung jeweils einer Zeile der Tabelle A entspricht

### Tabelle 22

Verbindungen der Formel I, in denen Q für C(=NOCH₃)-COOCH₃, R¹ und R² gemeinsam für Cyclopropyl stehen und die Kombination der Reste Y, X, X', R³ und R⁴ für eine Verbindung jeweils einer Zeile der Tabelle A entspricht

### Tabelle 23

Verbindungen der Formel I, in denen Q für C(=CHOCH₃)-COOCH₃, R¹ und R² gemeinsam für Cyclopropyl stehen und die Kombination der Reste Y, X, X', R³ und R⁴ für eine Verbindung jeweils einer Zeile der Tabelle A entspricht

### Tabelle 24

Verbindungen der Formel I, in denen Q für C(=CHCH₃)-COOCH₃, R¹ und R² gemeinsam für Cyclopropyl stehen und die Kombination der Reste Y, X, X', R³ und R⁴ für eine Verbindung jeweils einer Zeile der Tabelle A entspricht

### Tabelle 25

Verbindungen der Formel I, in denen Q für N(-OCH₃)-COOCH₃, R¹ und R² gemeinsam für Cyclopropyl stehen und die Kombination der Reste Y, X, X', R³ und R⁴ für eine Verbindung jeweils einer Zeile der Tabelle A entspricht

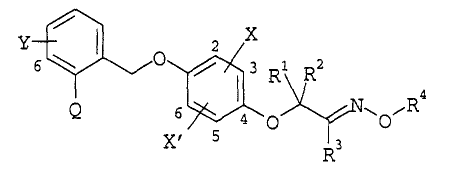

**Tabelle A**

| Nr. | Y | X | X' | R³ | R⁴ |
|---|---|---|---|---|---|
| A-1 | H | 2-CH₃ | H | CH₃ | CH₃ |
| A-2 | H | 2-CH₃ | 5-CH₃ | CH₃ | CH₃ |
| A-3 | H | 2-CH₃ | 5-Cl | CH₃ | CH₃ |
| A-4 | H | 2-CH₃ | 5-F | CH₃ | CH₃ |
| A-5 | H | 2-CH₃ | 5-CF₃ | CH₃ | CH₃ |
| A-6 | 6-CH₃ | 2-CH₃ | H | CH₃ | CH₃ |
| A-7 | 6-CH₃ | 2-CH₃ | 5-CH₃ | CH₃ | CH₃ |
| A-8 | 6-CH₃ | 2-CH₃ | 5-Cl | CH₃ | CH₃ |
| A-9 | 6-CH₃ | 2-CH₃ | 5-F | CH₃ | CH₃ |
| A-10 | 6-CH₃ | 2-CH₃ | 5-CF₃ | CH₃ | CH₃ |
| A-11 | 6-Cl | 2-CH₃ | H | CH₃ | CH₃ |
| A-12 | 6-Cl | 2-CH₃ | 5-CH₃ | CH₃ | CH₃ |
| A-13 | 6-Cl | 2-CH₃ | 5-Cl | CH₃ | CH₃ |
| A-14 | 6-Cl | 2-CH₃ | 5-F | CH₃ | CH₃ |
| A-15 | 6-Cl | 2-CH₃ | 5-CF₃ | CH₃ | CH₃ |
| A-16 | H | 2-CH₃ | H | CH₂CH₃ | CH₃ |
| A-17 | H | 2-CH₃ | 5-CH₃ | CH₂CH₃ | CH₃ |
| A-18 | H | 2-CH₃ | 5-Cl | CH₂CH₃ | CH₃ |
| A-19 | H | 2-CH₃ | 5-F | CH₂CH₃ | CH₃ |
| A-20 | H | 2-CH₃ | 5-CF₃ | CH₂CH₃ | CH₃ |
| A-21 | 6-CH₃ | 2-CH₃ | H | CH₂CH₃ | CH₃ |
| A-22 | 6-CH₃ | 2-CH₃ | 5-CH₃ | CH₂CH₃ | CH₃ |
| A-23 | 6-CH₃ | 2-CH₃ | 5-Cl | CH₂CH₃ | CH₃ |
| A-24 | 6-CH₃ | 2-CH₃ | 5-F | CH₂CH₃ | CH₃ |
| A-25 | 6-CH₃ | 2-CH₃ | 5-CF₃ | CH₂CH₃ | CH₃ |
| A-26 | 6-Cl | 2-CH₃ | H | CH₂CH₃ | CH₃ |
| A-27 | 6-Cl | 2-CH₃ | 5-CH₃ | CH₂CH₃ | CH₃ |
| A-28 | 6-Cl | 2-CH₃ | 5-Cl | CH₂CH₃ | CH₃ |
| A-29 | 6-Cl | 2-CH₃ | 5-F | CH₂CH₃ | CH₃ |
| A-30 | 6-Cl | 2-CH₃ | 5-CF₃ | CH₂CH₃ | CH₃ |
| A-31 | H | 2-CH₃ | H | CF₃ | CH₃ |
| A-32 | H | 2-CH₃ | 5-CH₃ | CF₃ | CH₃ |
| A-33 | H | 2-CH₃ | 5-Cl | CF₃ | CH₃ |
| A-34 | H | 2-CH₃ | 5-F | CF₃ | CH₃ |
| A-35 | H | 2-CH₃ | 5-CF₃ | CF₃ | CH₃ |
| A-36 | 6-CH₃ | 2-CH₃ | H | CF₃ | CH₃ |
| A-37 | 6-CH₃ | 2-CH₃ | 5-CH₃ | CF₃ | CH₃ |
| A-38 | 6-CH₃ | 2-CH₃ | 5-Cl | CF₃ | CH₃ |
| A-39 | 6-CH₃ | 2-CH₃ | 5-F | CF₃ | CH₃ |
| A-40 | 6-CH₃ | 2-CH₃ | 5-CF₃ | CF₃ | CH₃ |
| A-41 | 6-Cl | 2-CH₃ | H | CF₃ | CH₃ |
| A-42 | 6-Cl | 2-CH₃ | 5-CH₃ | CF₃ | CH₃ |
| A-43 | 6-Cl | 2-CH₃ | 5-Cl | CF₃ | CH₃ |
| A-44 | 6-Cl | 2-CH₃ | 5-F | CF₃ | CH₃ |
| A-45 | 6-Cl | 2-CH₃ | 5-CF₃ | CF₃ | CH₃ |
| A-46 | H | 3-CH₃ | H | CH₃ | CH₃ |
| A-47 | H | 3-CH₃ | 5-CH₃ | CH₃ | CH₃ |
| A-48 | H | 3-CH₃ | 5-Cl | CH₃ | CH₃ |
| A-49 | H | 3-CH₃ | 5-F | CH₃ | CH₃ |
| A-50 | H | 3-CH₃ | 5-CF₃ | CH₃ | CH₃ |
| A-51 | H | 3-CH₃ | H | CH₂CH₃ | CH₃ |
| A-52 | H | 3-CH₃ | 5-CH₃ | CH₂CH₃ | CH₃ |
| A-53 | H | 3-CH₃ | 5-Cl | CH₂CH₃ | CH₃ |
| A-54 | H | 3-CH₃ | 5-F | CH₂CH₃ | CH₃ |
| A-55 | H | 3-CH₃ | 5-CF₃ | CH₂CH₃ | CH₃ |
| A-56 | H | 3-CH₃ | H | CF₃ | CH₃ |
| A-57 | H | 3-CH₃ | 5-CH₃ | CF₃ | CH₃ |
| A-58 | H | 3-CH₃ | 5-Cl | CF₃ | CH₃ |
| A-59 | H | 3-CH₃ | 5-F | CF₃ | CH₃ |
| A-60 | H | 3-CH₃ | 5-CF₃ | CF₃ | CH₃ |
| A-61 | H | 2-CH₃ | H | CH₃ | CH₂CH₃ |
| A-62 | H | 2-CH₃ | 5-CH₃ | CH₃ | CH₂CH₃ |
| A-63 | H | 2-CH₃ | 5-Cl | CH₃ | CH₂CH₃ |
| A-64 | H | 2-CH₃ | 5-F | CH₃ | CH₂CH₃ |
| A-65 | H | 2-CH₃ | 5-CF₃ | CH₃ | CH₂CH₃ |
| A-66 | 6-CH₃ | 2-CH₃ | H | CH₃ | CH₂CH₃ |
| A-67 | 6-CH₃ | 2-CH₃ | 5-CH₃ | CH₃ | CH₂CH₃ |
| A-68 | 6-CH₃ | 2-CH₃ | 5-Cl | CH₃ | CH₂CH₃ |
| A-69 | 6-CH₃ | 2-CH₃ | 5-F | CH₃ | CH₂CH₃ |
| A-70 | 6-CH₃ | 2-CH₃ | 5-CF₃ | CH₃ | CH₂CH₃ |
| A-71 | 6-Cl | 2-CH₃ | H | CH₃ | CH₂CH₃ |
| A-72 | 6-Cl | 2-CH₃ | 5-CH₃ | CH₃ | CH₂CH₃ |
| A-73 | 6-Cl | 2-CH₃ | 5-Cl | CH₃ | CH₂CH₃ |
| A-74 | 6-Cl | 2-CH₃ | 5-F | CH₃ | CH₂CH₃ |
| A-75 | 6-Cl | 2-CH₃ | 5-CF₃ | CH₃ | CH₂CH₃ |
| A-76 | H | 2-CH₃ | H | CH₂CH₃ | CH₂CH₃ |
| A-77 | H | 2-CH₃ | 5-CH₃ | CH₂CH₃ | CH₂CH₃ |
| A-78 | H | 2-CH₃ | 5-Cl | CH₂CH₃ | CH₂CH₃ |
| A-79 | H | 2-CH₃ | 5-F | CH₂CH₃ | CH₂CH₃ |
| A-80 | H | 2-CH₃ | 5-CF₃ | CH₂CH₃ | CH₂CH₃ |
| A-81 | 6-CH₃ | 2-CH₃ | H | CH₂CH₃ | CH₂CH₃ |
| A-82 | 6-CH₃ | 2-CH₃ | 5-CH₃ | CH₂CH₃ | CH₂CH₃ |
| A-83 | 6-CH₃ | 2-CH₃ | 5-Cl | CH₂CH₃ | CH₂CH₃ |
| A-84 | 6-CH₃ | 2-CH₃ | 5-F | CH₂CH₃ | CH₂CH₃ |
| A-85 | 6-CH₃ | 2-CH₃ | 5-CF₃ | CH₂CH₃ | CH₂CH₃ |
| A-86 | 6-Cl | 2-CH₃ | H | CH₂CH₃ | CH₂CH₃ |
| A-87 | 6-Cl | 2-CH₃ | 5-CH₃ | CH₂CH₃ | CH₂CH₃ |
| A-88 | 6-Cl | 2-CH₃ | 5-Cl | CH₂CH₃ | CH₂CH₃ |
| A-89 | 6-Cl | 2-CH₃ | 5-F | CH₂CH₃ | CH₂CH₃ |
| A-90 | 6-Cl | 2-CH₃ | 5-CF₃ | CH₂CH₃ | CH₂CH₃ |
| A-91 | H | 2-CH₃ | H | CF₃ | CH₂CH₃ |
| A-92 | H | 2-CH₃ | 5-CH₃ | CF₃ | CH₂CH₃ |
| A-93 | H | 2-CH₃ | 5-Cl | CF₃ | CH₂CH₃ |
| A-94 | H | 2-CH₃ | 5-F | CF₃ | CH₂CH₃ |
| A-95 | H | 2-CH₃ | 5-CF₃ | CF₃ | CH₂CH₃ |
| A-96 | 6-CH₃ | 2-CH₃ | H | CF₃ | CH₂CH₃ |
| A-97 | 6-CH₃ | 2-CH₃ | 5-CH₃ | CF₃ | CH₂CH₃ |
| A-98 | 6-CH₃ | 2-CH₃ | 5-Cl | CF₃ | CH₂CH₃ |
| A-99 | 6-CH₃ | 2-CH₃ | 5-F | CF₃ | CH₂CH₃ |
| A-100 | 6-CH₃ | 2-CH₃ | 5-CF₃ | CF₃ | CH₂CH₃ |
| A-101 | 6-Cl | 2-CH₃ | H | CF₃ | CH₂CH₃ |
| A-102 | 6-Cl | 2-CH₃ | 5-CH₃ | CF₃ | CH₂CH₃ |
| A-103 | 6-Cl | 2-CH₃ | 5-Cl | CF₃ | CH₂CH₃ |
| A-104 | 6-Cl | 2-CH₃ | 5-F | CF₃ | CH₂CH₃ |
| A-105 | 6-Cl | 2-CH₃ | 5-CF₃ | CF₃ | CH₂CH₃ |
| A-106 | H | 2-CH₃ | H | CH₃ | CH₂CH=CH₂ |
| A-107 | H | 2-CH₃ | 5-CH₃ | CH₃ | CH₂CH=CH₂ |
| A-108 | H | 2-CH₃ | 5-Cl | CH₃ | CH₂CH=CH₂ |
| A-109 | H | 2-CH₃ | 5-F | CH₃ | CH₂CH=CH₂ |
| A-110 | H | 2-CH₃ | 5-CF₃ | CH₃ | CH₂CH=CH₂ |
| A-111 | 6-CH₃ | 2-CH₃ | H | CH₃ | CH₂CH=CH₂ |
| A-112 | 6-CH₃ | 2-CH₃ | 5-CH₃ | CH₃ | CH₂CH=CH₂ |
| A-113 | 6-CH₃ | 2-CH₃ | 5-Cl | CH₃ | CH₂CH=CH₂ |
| A-114 | 6-CH₃ | 2-CH₃ | 5-F | CH₃ | CH₂CH=CH₂ |
| A-115 | 6-CH₃ | 2-CH₃ | 5-CF₃ | CH₃ | CH₂CH=CH₂ |
| A-116 | 6-Cl | 2-CH₃ | H | CH₃ | CH₂CH=CH₂ |
| A-117 | 6-Cl | 2-CH₃ | 5-CH₃ | CH₃ | CH₂CH=CH₂ |
| A-118 | 6-Cl | 2-CH₃ | 5-Cl | CH₃ | CH₂CH=CH₂ |
| A-119 | 6-Cl | 2-CH₃ | 5-F | CH₃ | CH₂CH=CH₂ |
| A-120 | 6-Cl | 2-CH₃ | 5-CF₃ | CH₃ | CH₂CH=CH₂ |
| A-121 | H | 2-CH₃ | H | CH₂CH₃ | CH₂CH=CH₂ |
| A-122 | H | 2-CH₃ | 5-CH₃ | CH₂CH₃ | CH₂CH=CH₂ |
| A-123 | H | 2-CH₃ | 5-Cl | CH₂CH₃ | CH₂CH=CH₂ |
| A-124 | H | 2-CH₃ | 5-F | CH₂CH₃ | CH₂CH=CH₂ |
| A-125 | H | 2-CH₃ | 5-CF₃ | CH₂CH₃ | CH₂CH=CH₂ |
| A-126 | 6-CH₃ | 2-CH₃ | H | CH₂CH₃ | CH₂CH=CH₂ |
| A-127 | 6-CH₃ | 2-CH₃ | 5-CH₃ | CH₂CH₃ | CH₂CH=CH₂ |
| A-128 | 6-CH₃ | 2-CH₃ | 5-Cl | CH₂CH₃ | CH₂CH=CH₂ |
| A-129 | 6-CH₃ | 2-CH₃ | 5-F | CH₂CH₃ | CH₂CH=CH₂ |
| A-130 | 6-CH₃ | 2-CH₃ | 5-CF₃ | CH₂CH₃ | CH₂CH=CH₂ |
| A-131 | 6-Cl | 2-CH₃ | H | CH₂CH₃ | CH₂CH=CH₂ |
| A-132 | 6-Cl | 2-CH₃ | 5-CH₃ | CH₂CH₃ | CH₂CH=CH₂ |
| A-133 | 6-Cl | 2-CH₃ | 5-Cl | CH₂CH₃ | CH₂CH=CH₂ |
| A-134 | 6-Cl | 2-CH₃ | 5-F | CH₂CH₃ | CH₂CH=CH₂ |
| A-135 | 6-Cl | 2-CH₃ | 5-CF₃ | CH₂CH₃ | CH₂CH=CH₂ |
| A-136 | H | 2-CH₃ | H | CF₃ | CH₂CH=CH₂ |
| A-137 | H | 2-CH₃ | 5-CH₃ | CF₃ | CH₂CH=CH₂ |
| A-138 | H | 2-CH₃ | 5-Cl | CF₃ | CH₂CH=CH₂ |
| A-139 | H | 2-CH₃ | 5-F | CF₃ | CH₂CH=CH₂ |
| A-140 | H | 2-CH₃ | 5-CF₃ | CF₃ | CH₂CH=CH₂ |
| A-141 | 6-CH₃ | 2-CH₃ | H | CF₃ | CH₂CH=CH₂ |
| A-142 | 6-CH₃ | 2-CH₃ | 5-CH₃ | CF₃ | CH₂CH=CH₂ |
| A-143 | 6-CH₃ | 2-CH₃ | 5-Cl | CF₃ | CH₂CH=CH₂ |
| A-144 | 6-CH₃ | 2-CH₃ | 5-F | CF₃ | CH₂CH=CH₂ |
| A-145 | 6-CH₃ | 2-CH₃ | 5-CF₃ | CF₃ | CH₂CH=CH₂ |
| A-146 | 6-Cl | 2-CH₃ | H | CF₃ | CH₂CH=CH₂ |
| A-147 | 6-Cl | 2-CH₃ | 5-CH₃ | CF₃ | CH₂CH=CH₂ |
| A-148 | 6-Cl | 2-CH₃ | 5-Cl | CF₃ | CH₂CH=CH₂ |
| A-149 | 6-Cl | 2-CH₃ | 5-F | CF₃ | CH₂CH=CH₂ |
| A-150 | 6-Cl | 2-CH₃ | 5-CF₃ | CF₃ | CH₂CH=CH₂ |

| Nr . | Y | X | X' | R³ | R⁴ |
|---|---|---|---|---|---|
| A-151 | H | 2-CH₃ | H | CH₃ | CH₂C≡CH |
| A-152 | H | 2-CH₃ | 5-CH₃ | CH₃ | CH₂C≡CH |
| A-153 | H | 2-CH₃ | 5-Cl | CH₃ | CH₂C≡CH |
| A-154 | H | 2-CH₃ | 5-F | CH₃ | CH₂C≡CH |
| A-155 | H | 2-CH₃ | 5-CF₃ | CH₃ | CH₂C≡CH |
| A-156 | 6-CH₃ | 2-CH₃ | H | CH₃ | CH₂C≡CH |
| A-157 | 6-CH₃ | 2-CH₃ | 5-CH₃ | CH₃ | CH₂C≡CH |
| A-158 | 6-CH₃ | 2-CH₃ | 5-Cl | CH₃ | CH₂C≡CH |
| A-159 | 6-CH₃ | 2-CH₃ | 5-F | CH₃ | CH₂C≡CH |
| A-160 | 6-CH₃ | 2-CH₃ | 5-CF₃ | CH₃ | CH₂C≡CH |
| A-161 | 6-Cl | 2-CH₃ | H | CH₃ | CH₂C≡CH |
| A-162 | 6-Cl | 2-CH₃ | 5-CH₃ | CH₃ | CH₂C≡CH |
| A-163 | 6-Cl | 2-CH₃ | 5-Cl | CH₃ | CH₂C≡CH |
| A-164 | 6-Cl | 2-CH₃ | 5-F | CH₃ | CH₂C≡CH |
| A-165 | 6-Cl | 2-CH₃ | 5-CF₃ | CH₃ | CH₂C≡CH |
| A-166 | H | 2-CH₃ | H | CH₂CH₃ | CH₂C≡CH |
| A-167 | H | 2-CH₃ | 5-CH₃ | CH₂CH₃ | CH₂C≡CH |
| A-168 | H | 2-CH₃ | 5-Cl | CH₂CH₃ | CH₂C≡CH |
| A-169 | H | 2-CH₃ | 5-F | CH₂CH₃ | CH₂C≡CH |
| A-170 | H | 2-CH₃ | 5-CF₃ | CH₂CH₃ | CH₂C≡CH |
| A-171 | 6-CH₃ | 2-CH₃ | H | CH₂CH₃ | CH₂C≡CH |
| A-172 | 6-CH₃ | 2-CH₃ | 5-CH₃ | CH₂CH₃ | CH₂C≡CH |
| A-173 | 6-CH₃ | 2-CH₃ | 5-Cl | CH₂CH₃ | CH₂C≡CH |
| A-174 | 6-CH₃ | 2-CH₃ | 5-F | CH₂CH₃ | CH₂C≡CH |
| A-175 | 6-CH₃ | 2-CH₃ | 5-CF₃ | CH₂CH₃ | CH₂C≡CH |
| A-176 | 6-Cl | 2-CH₃ | H | CH₂CH₃ | CH₂C≡CH |
| A-177 | 6-Cl | 2-CH₃ | 5-CH₃ | CH₂CH₃ | CH₂C≡CH |
| A-178 | 6-Cl | 2-CH₃ | 5-Cl | CH₂CH₃ | CH₂C≡CH |
| A-179 | 6-Cl | 2-CH₃ | 5-F | CH₂CH₃ | CH₂C≡CH |
| A-180 | 6-Cl | 2-CH₃ | 5-CF₃ | CH₂CH₃ | CH₂C≡CH |
| A-181 | H | 2-CH₃ | H | CF₃ | CH₂C≡CH |
| A-182 | H | 2-CH₃ | 5-CH₃ | CF₃ | CH₂C≡CH |
| A-183 | H | 2-CH₃ | 5-Cl | CF₃ | CH₂C≡CH |
| A-184 | H | 2-CH₃ | 5-F | CF₃ | CH₂C≡CH |
| A-185 | H | 2-CH₃ | 5-CF₃ | CF₃ | CH₂C≡CH |
| A-186 | 6-CH₃ | 2-CH₃ | H | CF₃ | CH₂C≡CH |
| A-187 | 6-CH₃ | 2-CH₃ | 5-CH₃ | CF₃ | CH₂C≡CH |
| A-188 | 6-CH₃ | 2-CH₃ | 5-Cl | CF₃ | CH₂C≡CH |
| A-189 | 6-CH₃ | 2-CH₃ | 5-F | CF₃ | CH₂C≡CH |

| Nr. | Y | X | X' | R³ | R⁴ |
|---|---|---|---|---|---|
| A-190 | 6-CH₃ | 2-CH₃ | 5-CF₃ | CF₃ | CH₂C≡CH |
| A-191 | 6-Cl | 2-CH₃ | H | CF₃ | CH₂C≡CH |
| A-192 | 6-Cl | 2-CH₃ | 5-CH₃ | CF₃ | CH₂C≡CH |
| A-193 | 6-Cl | 2-CH₃ | 5-Cl | CF₃ | CH₂C≡CH |
| A-194 | 6-Cl | 2-CH₃ | 5-F | CF₃ | CH₂C≡CH |
| A-195 | 6-Cl | 2-CH₃ | 5-CF₃ | CF₃ | CH₂C≡CH |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten*, *Phycomyceten* und *Basidiomyceten*, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit.10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfid, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl) -phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis- (dimethylamino)phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl) -2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid;
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4- (2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan;
- Amine wie 2,6-Dimethyl-N-tridecyl-morpholin bzw. dessen Salze, 2,6-Dimethyl-N-cyclododecyl-morpholin bzw. dessen Salze, N-[3- (p-tert.-Butylphenyl) -2-methylpropyl] -cis-2,6-dimethylmorpholin, N-[3-(p-tert.-Butylphenyl)-2-methylpropyl]-piperidin, (8-(1,1-Dimethylethyl)-N-ethyl-N-propyl-1,4-dioxaspiro[4.5]decan-2-methanamin;
- Azole wie 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl) -N- (2,4,6-trichlorphenoxyethyl) -N'-imidazol-yl-harnstoff, 1-(4-Chlorphenoxy) -3,3-dimethyl-1-(1H-1,2,4-triazol-1-yl) -2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, 1-[2-(2,4-Dichlorphenyl)-pentyl]-1H-1,2,4-triazol, 2,4'-Difluor-a-(1H-1,2,4-triazolyl-1-methyl) -benzhydrylalkohol, 1-((bis- (4-Fluorphenyl)-methylsilyl)-methyl)-1H-1,2,4-triazol, 1-[(2RS,4RS;2RS,4SR) -4-brom-2- (2,4-dichlorphenyl) tetrahydrofuryl]-1H-1,2,4-triazol, 2-(4-Chlorphenyl)-3-cyclopropyl-1-(1H-1,2,4-triazol-1-yl)-butan-2-ol, (+)-4-Chlor-4-[4-methyl-2-(1H-1,2,4-triazol-1-ylmethyl)-1,3-dioxolan-2-yl] -phenyl-4-chlorphenylether, (E)-(R,S)-1-(2,4-dichlorphenyl)-4,4-dimethyl-2-(1H-1,2,4-triazol-1-yl)pent-1-en-3-ol, 4-(4-Chlorphenyl)-2-phenyl-2-(1H-1,2,4-triazolylmethyl)-butyronitril, 3-(2,4-dichlorphenyl) -6-fluor-2-(1H-1,2,4-triazol-1-yl) chinazolin-4(3H)-on, (R,S)-(2-(2,4-Dichlorphenyl)-1H-1,2,4-triazol-1-yl)-hexan-2-ol, (1RS,5RS;1RS,5SR)-5-(4-chlorbenzyl)-2,2-dimethyl-1-(1H-1,2,4-triazol-1-ylmethyl)cyclopentanol, (R,S)-1-(4-chlorphenyl)-4,4-dimethyl-3-(1H-1,2,4-triazol-1-ylmethyl)pentan-3-ol, (+)-2-(2,4-Dichlorphenyl)-3-(1H-1,2,4-triazolyl)-propyl-1,1,2,2-tetrafluorethylether, (E)-{1-[1- (4-Chlor-2-trifluormethyl)-phenyl] -imino-2-propoxyethyl)-1H-imidazol, 2- (4-Chlorphenyl)-2-(1H-1,2,4-triazol-1-ylmethyl)-hexannitril;
- α-(2-Chlorphenyl)-α-(4-chlorphenyl) -5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol;
- Strobilurine wie Methyl-E-methoxyimino*-*[*α-*(o-tolyloxy) -o-tolyl]acetat, Methyl-E-2-{2-[6-(2-cyanophenoxy)-pyrimidin-4-yloxy]-phenyl}-3-methoxyacrylat, Methyl-E-methoxyimino-[α- (2-phenoxyphenyl)]-acetamid, Methyl-E-methoxyimino-[α-(2,5-dimethylphenoxy)-o-tolyl] -acetamid,
- Methyl-E-2-{2-[2-trifluormethylpyridyl-6-]oxymethyl]phenyl}3-methoxyacrylat, (E,E) -Methoximino-{2-[1-(3-trifluormethylphenyl) -ethylidenaminooxymethyl]-phenyl}essigsäuremethylester;
- Methyl-N-(2-{[1-(4-chlorophenyl)-1H-pyrazol-3-yl]oxymethyl}phenyl) N-methoxycarbamat;
- Anilinopyrimidine wie N- (4,6-Dimethylpyrimidin-2-yl) -anilin, N- [4-Methyl-6- (1-propinyl) -pyrimidin-2-yl] -anilin, N-[4-Methyl-6-cyclopropyl-pyrimidin-2-yl]-anilin;
- Phenylpyrrole wie 4-(2,2-Difluor-1,3-benzodioxol-4-yl)-pyrrol-3-carbonitril;
- Zimtsäureamide wie 3-(4-Chlorphenyl)-3-(3,4-dimethoxyphenyl)-acrylsäuremorpholid;
- sowie verschiedene Fungizide, wie Dodecylguanidinacetat, 3-[3-(3,5-Dimethyl-2-oxycyclohexyl)-2-hydroxyethyl]-glutarimid, N-Methyl-N-ethyl- (4-trifluormethyl,-2- [3',4'-dimethoxyphenyl]-benzoesäureamid, Hexachlorbenzol, DL-Methyl-N-(2,6-dimethyl-phenyl)-N-furoyl(2)-alaninat, DL-N- (2,6-Dimethyl-phenyl)-N-(2'-methoxyacetyl) -alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl) -2,4-dioxo-1,3-oxazolidin, 3-(3,5-Dichlorphenyl) -5-methyl-5-methoxymethyl-1,3-oxazolidin-2,4-dion, 3- (3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, N-(3-Chlor-2,6-dinitro-4-trifluormethyl-phenyl)-5-trifluormethyl-3-chlor-2-aminopyridin.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1 Herstellung von [2-(4-Acetoxy-2-methyl-phenoxymethyl) -phenyl] -methoxyimino-essigsäuremethylester

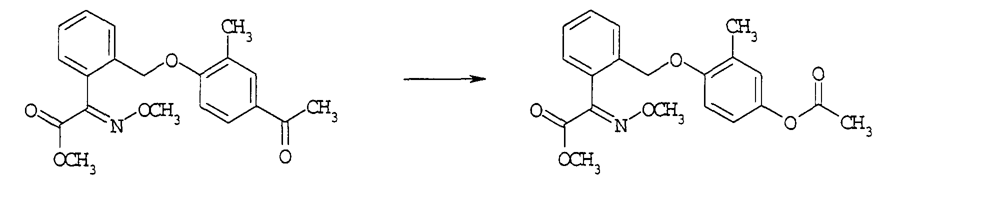

Eine Lösung von 106,1 g E-2-[(2'-Methyl-4'-acetyl)-Phenoxymethyl] -phenylglyoxylsäuremethylester-O-methyloxim [vgl. EP-A 621 264] in 240 ml CH₂Cl₂ wurde nach Versetzen mit 188,3 g 3-Chlorperbenzoesäure (MCPBA) etwa 3 Tage bei 20-25°C gerührt. Nach Abfiltrieren des Niederschlags und Waschen der organischen Phasen mit ges. NaS₂O₃- und NaHCO₃-Lösungen und Wasser wurde das Lösungsmittel abdestilliert. Aus dem Rückstand erhielt man 104 g der Titelverbindung als hellbraune kristalline Substanz vom Schmp. 108-110°C.
¹H-NMR (δ [CDCl₃]): 2,2(s,3H); 2,3(s,3H); 3,8(s,3H); 4,0(s,3H); 5,0(s,3H); 6,7-7,0(m,3H); 7,2-7,6(m,4H).

### Beispiel 2 Herstellung von [2-(4-Hydroxy-2-methyl-phenoxymethyl) -phenyl] -methoxyimino-essigsäuremethylester

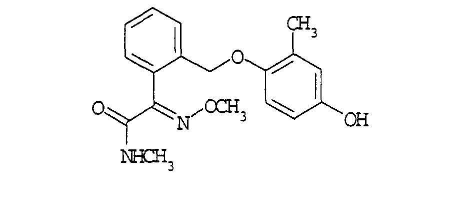

Eine Lösung von 50,7 g der Verbindung aus Beispiel 1 in 80 ml Tetrahydrofuran (THF) wurde nach Versetzen mit 42 g 40%iger wässriger Methylamin-Lösung etwa 12 Std. bei 20-25°C gerührt. Nach Abdestillieren des Lösungsmittels und Aufnehmen des Rückstandes in Methyl-tert. Butylether/Wasser-Gemisch wurden die Phasen getrennt. Die organische Phase wurde nach Trocknen vom Lösungsmittel befreit. Aus dem Rückstand erhielt man nach Digerieren mit Diisopropylether 40,6 g der Titelverbindung als hellbraune kristalline Substanz vom Schmp. 148-150°C.
¹H-NMR (δ [CDCl₃]): 2,2(s,3H); 3,0(d,3H); 4,0(s,3H); 5,0(s,2H); 4,6(s,1H); 6,6(m,1H); 6,7-7,6(m,7H).

### Beispiel 3 Herstellung von 2-Methoxyimino-N-methyl-2-{2- [2-methyl-4- (1-methyl-2-oxo-propoxy)-phenoxymethyl] -phenyl}-acetamid

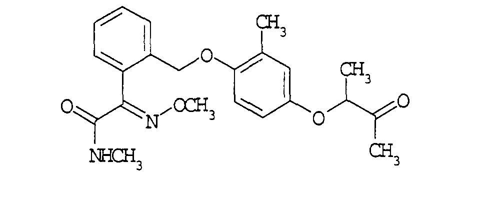

Eine Lösung von 22 g der Verbindung aus Beispiel 2 in 200 ml Dimethylformamid (DMF) wurde nach Versetzen mit 50 g K₂CO₃ und 8,5 g 3-Chlor-2-butanon 5 Std. bei etwa 50°C gerührt. Nach Abdestillieren des Lösungsmittels und Aufnehmen des Rückstandes in Methyl-tert. Butylether/Wasser-Gemisch wurden die Phasen getrennt. Die organische Phase wurde nach Trocknen vom Lösungsmittel befreit. Aus dem Rückstand erhielt man 21,7 g der Titelverbindung als braunes Öl.
¹H-NMR (δ [CDCl₃]): 1,4(d,3H); 1,18(s,3H); 1,2(s,3H); 3,0(d,3H); 4,0 (3H); 4,5(q,1H); 5,0(s,2H).

### Beispiel 4 Herstellung von 2-Methoxyimino-2-{2-[4-(2-methoxyimino-1-methyl-propoxy)-2-methyl-phenoxymethyl]-phenyl}-N-methyl-acetamid [I-1]

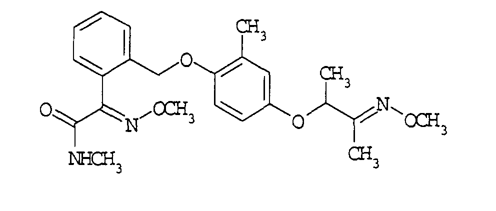

Eine Lösung von 21,7 g der Verbindung aus Beispiel 3 in 100 ml Methanol wurde nach Versetzen mit 30,4 g 30 %iger wässr. Methoxyamin-Lösung 12 Std. bei 20-25°C gerührt. Nach Abdestillieren des Lösungsmittels und Aufnehmen des Rückstandes in Methyl-tert. Butylether/Wasser-Gemisch wurden die Phasen getrennt. Die organische Phase wurde nach Trocknen vom Lösungsmittel befreit. Aus dem Rückstand erhielt man 22 g der Titelverbindung als hellbraunes Öl.
¹H-NMR (δ [CDCl₃]): 1,4(d,3H); 1,8(s,3H); 2,8(d,3H); 3,8(s,3H); 4,0(s,3H); 4,8(q,1H); 5,0(s,2H).

### Beispiel 5 Herstellung von [2-(4-Hydroxy-2-methyl-phenoxymethyl)-phenyl]-methoxyimino-essigsäuremethylester

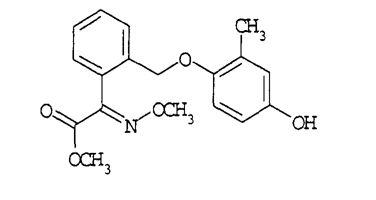

Eine Lösung von 50,7 g der Verbindung aus Beispiel 1 in 15 ml 30 %iger methanolischer Natriummethanolat-Lösung wurde 2 Std. refluxiert. Nach Abdestillieren des Lösungsmittels und Aufnehmen des Rückstandes in CH₂Cl₂/NH₄Cl-Lösung-Gemisch wurden die Phasen getrennt. Die organische Phase wurde nach Waschen mit NH₄Cl-Lösung und Trocknen vom Lösungsmittel befreit. Aus dem Rückstand erhielt man 39,2 g der Titelverbindung als hellbraune kristalline Substanz vom Schmp. 143-146°C.
¹H-NMR (δ [CDCl₃]): 2,2(s,3H); 3,8(s,3H); 4,0(s,3H); 4,6(s,1H[OH]); 4,9(s,2H).

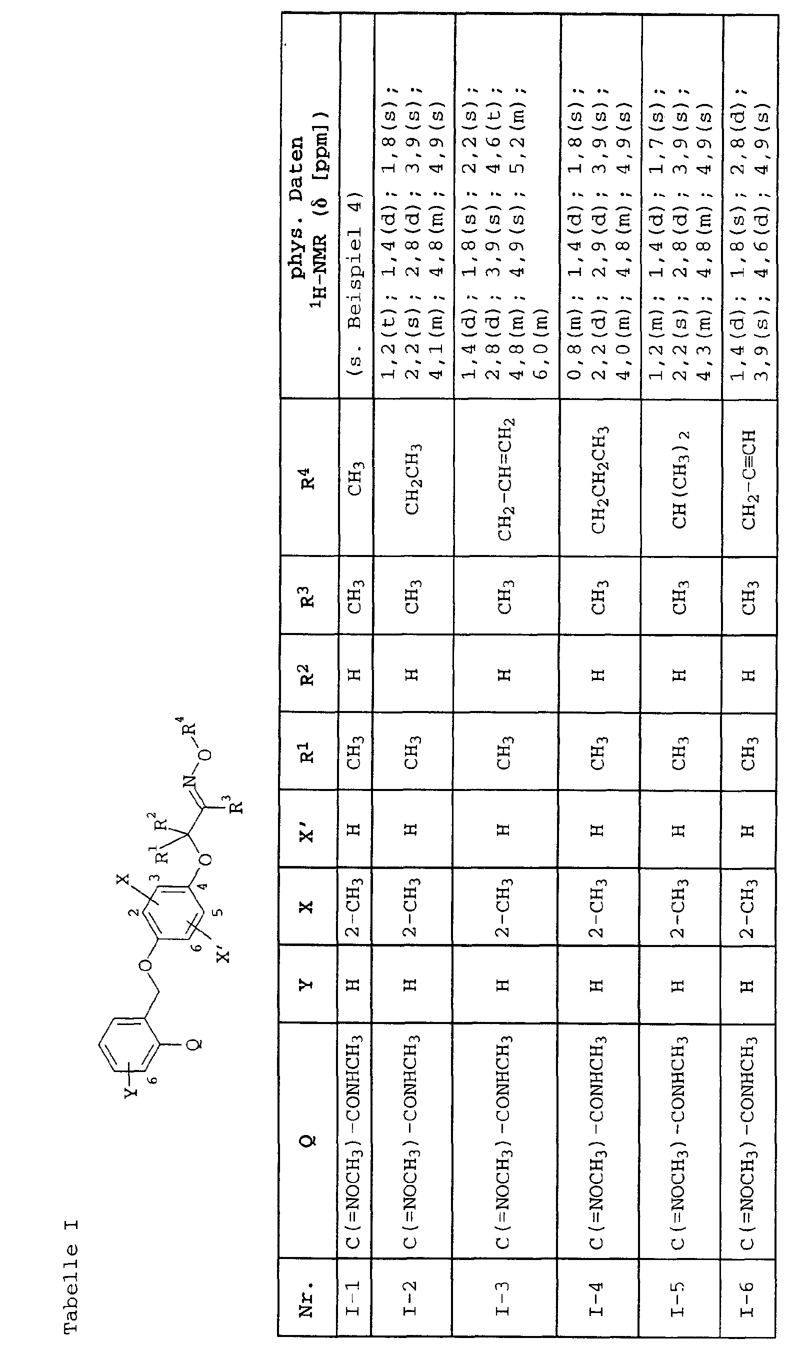

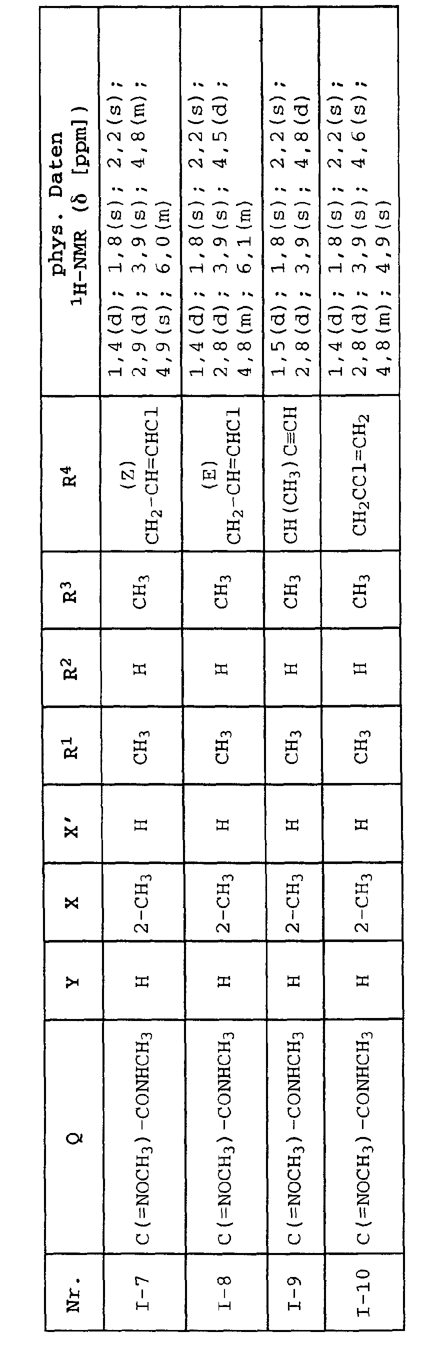

### Beispiele für die Wirkung gegen Schadpilze

Die fungizide Wirkung der Verbindungen der Formel I ließ sich durch die folgenden Versuche zeigen:

Die Wirkstoffe wurden als 10%ige Emulsion in einem Gemisch aus 63 Gew.-% Cyclohexanon und 27 Gew.-% Emulgator aufbereitet und entsprechend der gewünschten Konzentration mit Wasser verdünnt.

Als Vergleichswirkstoffe dienten die aus EP-A 477 631 als Nr.415 bekannte Verbindung A und die aus EP-A 579 124 als Nr. 1.007 bekannte Verbindung B:

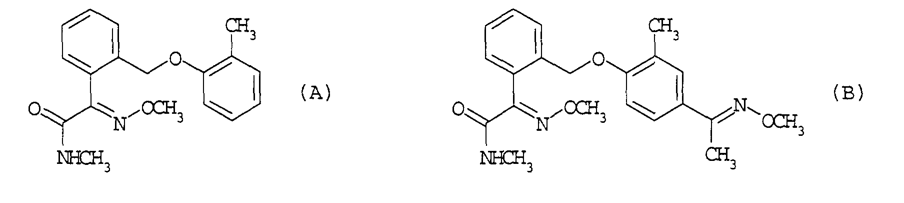

### Anwendungsbeispiel 1 - Wirksamkeit gegen Weizenmehltau

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Erysiphe graminis forma specialis tritici) bestäubt. Die Versuchspflanzen wurden anschließend im Gewächshaus bei Temperaturen zwischen 20 und 24 °C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

In diesem Test zeigten die mit 1 ppm des Wirkstoffs I-1 der Tabelle I behandelten Pflanzen keinen Befall, während die mit 1 ppm der Vergleichswirkstoffe A, bzw. B behandelten Pflanzen zu 60 %, bzw. 25 % und die unbehandelten Pflanzen zu 90 % befallen waren.

Anwendungsbeispiel 2 - Kurative Wirksamkeit gegen Puccinia recondita an Weizen (Weizenbraunrost)

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden in eine Kammer mit hoher Luftfeuchtigkeit (90 bis 95 %) und 20 bis 22 °C gestellt. Während dieser Zeit keimten die Sporen aus und die Keimschläuche drangen in das Blattgewebe ein. Die infizierten Pflanzen wurden am nächsten Tag mit einer wäßrigen Wirkstoffaufbereitung, die aus einer Stammlösung bestehend aus 10 % Wirkstoff, 63 % Cyclohexanon und 27 % Emulgiermittel angesetzt worden war, tropfnaß besprüht. Nach dem Antrocknen des Spritzbelages wurden die Versuchspflanzen im Gewächshaus bei Temperaturen zwischen 20 und 22 °C und 65 bis 70 % relativer Luftfeuchte für 7 Tage kultiviert. Dann wurde das Ausmaß der Rostpilzentwicklung auf den Blättern ermittelt.

In diesem Test zeigten die mit 16 ppm der Wirkstoffe I-1 bis I-10 der Tabelle I behandelten Pflanzen keinen Befall, während die mit 16 ppm der Vergleichswirkstoffe A, bzw. B behandelten Pflanzen zu 60 %, bzw. 25 % und die unbehandelten Pflanzen zu 95 % befallen waren.

### Anwendungsbeispiel 3 - Wirksamkeit gegen Pyricularia oryzae (protektiv)

Blätter von in Töpfen gewachsenen Reiskeimlingen der Sorte "Tai-Nong 67" wurden mit wässriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Am folgenden Tag wurden die Pflanzen mit einer wäßrigen Sporensuspension von Pyricularia oryzae inokuliert. Anschließend wurden die Versuchspflanzen in Klimakammern bei 22 bis 24 °C und 95 bis 99 % relativer Luftfeuchtigkeit für 6 Tage aufgestellt. Dann wurde das Ausmaß der Befallsentwicklung auf den Blättern visuell ermittelt.

In diesem Test zeigten die mit 63 ppm der Wirkstoffe I-1 bis 1-10 der Tabelle I behandelten Pflanzen keinen Befall, während die mit 63 ppm der Vergleichswirkstoffe A, bzw. B behandelten Pflanzen zu 25 %, bzw. 40 % und die unbehandelten Pflanzen zu 85 % befallen waren.

## Patentansprüche

1. Iminooxisubstituierte Benzylphenylether der Formel I, 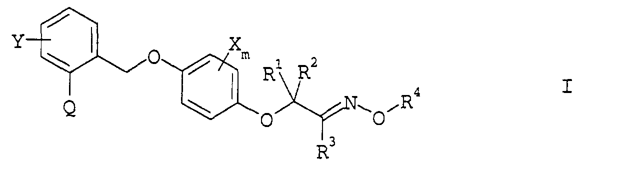 in der die Substituenten und der Index die folgenden Bedeutungen haben:
Y H, CH₃, F oder Cl;
Q C(=CHOCH₃) -COOCH₃, C(=CHCH₃) -COOCH₃, C(=NOCH₃) -COOCH₃, C(=NOCH₃)-CONHCH₃ oder N(-OCH₃)-COOCH₃;
x Wasserstoff, Halogen, C₁-C₄-Alkyl, C₁-C₄-Alkoxy oder CF₃;
m 1 oder 2, wobei die Reste X verschieden sein können, wenn m = 2 ist;
R¹ C₁-C₄-Alkyl und
R² Wasserstoff oder C₁-C₄-Alkyl; oder
R¹ und R² gemeinsam Cyclopropyl, Cyclopentyl oder Cyclohexyl;
R³ C₁-C₆-Alkyl oder CF₃;
R⁴ C₁-C₄-Alkyl, C₃-C₄-Alkenyl, C₃-C₄-Alkinyl, C₁-C₄-Halogenalkyl, C₃-C₄-Halogenalkenyl oder C3-C4-Halogenalkinyl;
sowie deren Salze.

2. Iminooxisubstituierte Benzylphenylether der Formel I' 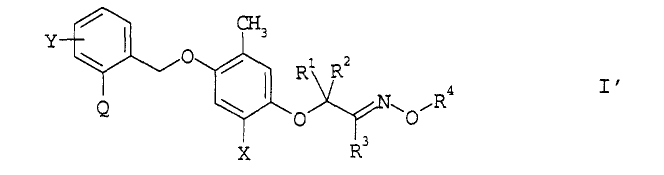 nach Anspruch 1, in denen X für Wasserstoff, Methyl, Chlor oder Fluor steht.

3. Verbindungen der Formel I' nach Anspruch 2, in denen Y in der 6-Position steht, X Wasserstoff bedeutet und R¹ für Methyl, Ethyl, iso-Propyl oder Cyclopropyl, R² für Wasserstoff steht und R³ Methyl oder Ethyl bedeutet.

4. Iminooxisubstituierte Benzylphenylether der Formel I'' 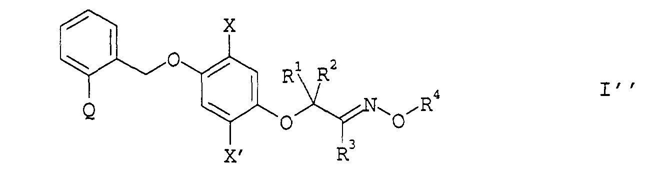 nach Ansprüchen 1 oder 2.

5. Verbindungen der Formel I" nach Anspruch 4, in denen X und R¹ für Methyl stehen und X' und R² Wasserstoff bedeutet.

6. Verfahren zur Herstellung der Verbindungen der Formel I nach Anspruch 1, dadurch gekennzeichnet, daß man Acetylverbindungen der Formel II.1 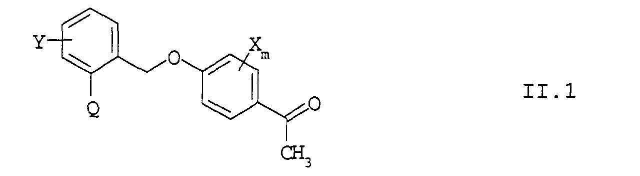 zu O-Acetylverbindungen II.2 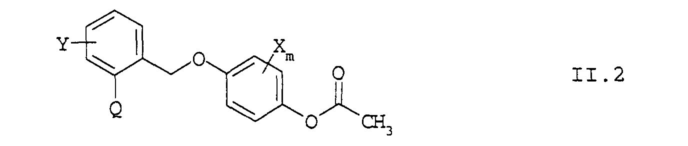 oxidiert, die unter basischen Bedingungen zu Hydroxiverbindungen II 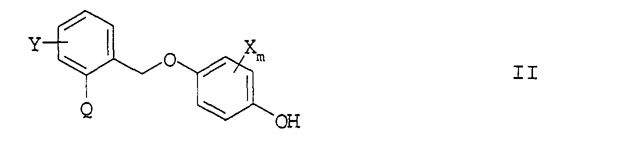 umgesetzt werden, II mit Ketoverbindungen der Formel III, 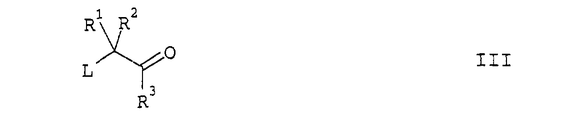 in der L für eine nucleophil austauschbare Gruppe steht, zu Verbindungen der Formel IV 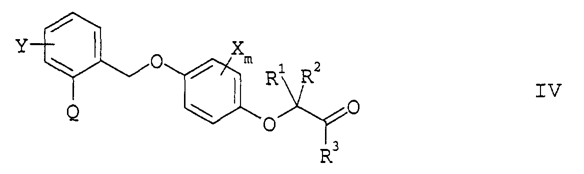 umsetzt und IV mit Aminoverbindungen der Formel V
H₂N-OR⁴ V
oder den entsprechenden Säureadditionssalzen zu I oximiert.

7. Verfahren gemäß Anspruch 6 zur Herstellung von Verbindungen der Formel I, in denen Q für C(=NOCH₃)-CONHCH₃ steht, dadurch gekennzeichnet, daß Verbindungen der Formel II.1' 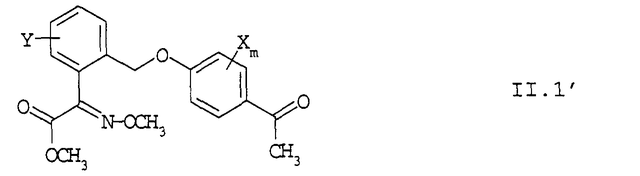 zu O-Acetylverbindungen II.2' 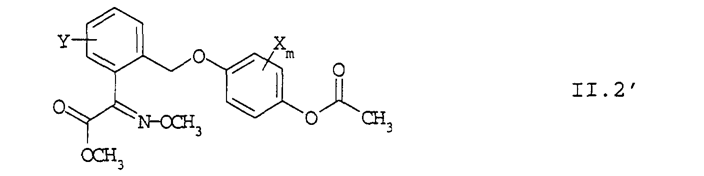 oxidiert werden, die mit Methylamin zu Hydroxiverbindungen II, in denen Q für C(=NOCH₃)-CONHCH₃ steht, umgesetzt werden.

8. Zwischenprodukte der Formel II gemäß Anspruch 6.

9. Zwischenprodukte der Formel IV gemäß Anspruch 6.

10. Zur Bekämpfung von Schadpilzen geeignetes Mittel, enthaltend einen festen oder flüssigen Trägerstoff und eine Verbindung der Formel I gemäß Anspruch 1.

11. Verfahren zur Bekämpfung von Schadpilzen, dadurch gekennzeichnet, daß man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.
